(19) Europäisches Patentamt / European Patent Office / Office européen des brevets

(11) **EP 3 316 949 B1**

(12) **EUROPEAN PATENT SPECIFICATION**

(45) Date of publication and mention of the grant of the patent:
**17.11.2021 Bulletin 2021/46**

(21) Application number: **16733587.6**

(22) Date of filing: **30.06.2016**

(51) Int Cl.:
**A61M 16/00** (2006.01)

(86) International application number:
**PCT/EP2016/065384**

(87) International publication number:
**WO 2017/001601 (05.01.2017 Gazette 2017/01)**

(54) **BAROMETRIC PRESSURE SENSOR FOR VARIABLE RESISTANCE POSITIVE AIRWAY PRESSURE DEVICE CIRCUIT COMPENSATION**

LUFTDRUCKFÜHLER FÜR SCHALTKREISKOMPENSATION EINER POSITIVATEMWEGDRUCKVORRICHTUNG MIT VARIABLEN WIDERSTAND

CAPTEUR DE PRESSION BAROMÉTRIQUE POUR COMPENSATION DE CIRCUIT DE DISPOSITIF DE VENTILATION SPONTANÉE EN PRESSION POSITIVE À RÉSISTANCE VARIABLE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2015 US 201562186431 P**

(43) Date of publication of application:
**09.05.2018 Bulletin 2018/19**

(73) Proprietor: **Koninklijke Philips N.V.**
**5656 AG Eindhoven (NL)**

(72) Inventor: **SHELLY, Benjamin, Irwin**
**5656 AE Eindhoven (NL)**

(74) Representative: **Philips Intellectual Property & Standards**
**High Tech Campus 5**
**5656 AE Eindhoven (NL)**

(56) References cited:
WO-A2-2014/057457    US-A- 5 881 717
US-A1- 2009 241 952    US-A1- 2010 078 023
US-A1- 2012 227 738    US-A1- 2013 056 006

## Description

BACKGROUND OF THE INVENTION

1. Field of the Invention

The present invention pertains to airway pressure support devices, and, in particular, to a positive airway pressure support device that employs a barometric pressure sensor in the airflow path to compensate for variable resistance in the patient circuit.

2. Description of the Related Art

Many individuals suffer from disordered breathing during sleep. Sleep apnea is a common example of such sleep disordered breathing suffered by millions of people throughout the world. One type of sleep apnea is obstructive sleep apnea (OSA), which is a condition in which sleep is repeatedly interrupted by an inability to breathe due to an obstruction of the airway; typically the upper airway or pharyngeal area. Obstruction of the airway is generally believed to be due, at least in part, to a general relaxation of the muscles which stabilize the upper airway segment, thereby allowing the tissues to collapse the airway. Another type of sleep apnea syndrome is a central apnea, which is a cessation of respiration due to the absence of respiratory signals from the brain's respiratory center. An apnea condition, whether obstructive, central, or mixed, which is a combination of obstructive and central, is defined as the complete or near cessation of breathing, for example a 90% or greater reduction in peak respiratory airflow.

Those afflicted with sleep apnea experience sleep fragmentation and complete or nearly complete cessation of ventilation intermittently during sleep with potentially severe degrees of oxyhemoglobin desaturation. These symptoms may be translated clinically into extreme daytime sleepiness, cardiac arrhythmias, pulmonary-artery hypertension, congestive heart failure and/or cognitive dysfunction. Other consequences of sleep apnea include right ventricular dysfunction, carbon dioxide retention during wakefulness, as well as during sleep, and continuous reduced arterial oxygen tension. Sleep apnea sufferers may be at risk for excessive mortality from these factors as well as by an elevated risk for accidents while driving and/or operating potentially dangerous equipment.

Even if a patient does not suffer from a complete or nearly complete obstruction of the airway, it is also known that adverse effects, such as arousals from sleep, can occur where there is only a partial obstruction of the airway. Partial obstruction of the airway typically results in shallow breathing referred to as a hypopnea. A hypopnea is typically defined as a 50% or greater reduction in the peak respiratory airflow. Other types of sleep disordered breathing include, without limitation, upper airway resistance syndrome (UARS) and vibration of the airway, such as vibration of the pharyngeal wall, commonly referred to as snoring.

It is well known to treat sleep disordered breathing by applying a continuous positive air pressure (CPAP) to the patient's airway. This positive pressure effectively "splints" the airway, thereby maintaining an open passage to the lungs. It is also known to provide a positive pressure therapy in which the pressure of gas delivered to the patient varies with the patient's breathing cycle, or varies with the patient's breathing effort, to increase the comfort to the patient. This pressure support technique is referred to as bi-level pressure support, in which the inspiratory positive airway pressure (IPAP) delivered to the patient is higher than the expiratory positive airway pressure (EPAP). It is further known to provide a positive pressure therapy in which the pressure is automatically adjusted based on the detected conditions of the patient, such as whether the patient is experiencing an apnea and/or hypopnea. This pressure support technique is referred to as an auto-titration type of pressure support, because the pressure support device seeks to provide a pressure to the patient that is only as high as necessary to treat the disordered breathing.

Pressure support therapies as just described involve the placement of a patient interface device including a mask component having a soft, flexible sealing cushion on the face of the patient. The mask component may be, without limitation, a nasal mask that covers the patient's nose, a nasal/oral mask that covers the patient's nose and mouth, or a full face mask that covers the patient's face. Such patient interface devices may also employ other patient contacting components, such as forehead supports, cheek pads and chin pads. The patient interface device is typically secured to the patient's head by a headgear component. The patient interface device is connected to a gas delivery tube or conduit to form what is commonly referred to as a patient circuit. The patient circuit interfaces the pressure support device with the airway of the patient, so that a flow of breathing gas can be delivered from the pressure/flow generating device to the airway of the patient.

Patient circuits as just described have pneumatic resistance (which is a function of flow) that needs to be compensated for with additional pressure by the pressure support device. In applications desiring a high degree of pressure delivery accuracy and/or with high or variable patient circuit resistance, monitoring pressure at the interface to the patient (i.e., proximal to the patient) is necessary so that the proper amount of additional compensation pressure can be determined and provided. For this purpose, it is known to the art to measure gauge pressure at the patient using a gauge pressure sensor located either at the patient (i.e., at the patient interface device) or inside the pressure support device (in which case gas is communicated from the patient interface device to the gauge pressure sensor through a pneumatic pick off tube).

Gauge pressure sensors are expensive and typically very large. In addition, power consumption is typically relatively high with gauge pressure sensors. US 2013/056006 discloses a system and method for delivering a flow of gas to an airway of a patient. WO 2014/057457 discloses a system and method for providing respiratory therapy through a pressure support device. US 2009/241952 discloses a breathing assistance system to determine pressure drop including a gas delivery system configured to deliver gas toward a patient, one or more sensors configured to measure one or more parameters of gas delivered by the gas delivery system, a calibration module, and a pressure drop calculation module. US 2012/227738 discloses a CPAP system including a flow generator, a patient interface, and an air delivery conduit. US 2010/078023 discloses a breathing assistance system for providing breathing assistance to a patient.

SUMMARY OF THE INVENTION

The invention is defined by the appended claims. Accordingly, it is an object of the present invention to provide a pressure support system that overcomes the shortcomings of conventional pressure support systems. This object is achieved according to one embodiment of the present disclosure by providing a pressure support system for delivering a flow of breathing gas to an airway of a patient that includes a pressure generating system structured to generate the flow of breathing gas, a patient circuit including a patient interface device, the patient circuit being coupled to the pressure generating system and structured to carry the flow of breathing gas, a barometric pressure sensor coupled to the patient circuit within a gas flow path of the patient circuit, wherein the barometric pressure sensor is structured to generate a barometric pressure signal indicative of a barometric pressure within the gas flow path proximate the patient, and a control system. The control system is structured to control an outlet pressure of the pressure generating system based on at least the barometric pressure signal in a manner that compensates for a pressure drop across the patient circuit due to a pneumatic resistance of the patient circuit.

It is yet another object of the present disclosure to provide a method of controlling a pressure support system that does not suffer from the disadvantages associated with conventional control techniques, the method not being part of the invention. This object is achieved by providing a method that includes determining a barometric pressure within a gas flow path of a patient circuit proximate the patient, and controlling an outlet pressure of a pressure generating system based on at least the barometric pressure in a manner that compensates for a pressure drop across the patient circuit due to a pneumatic resistance of the patient circuit.

These and other objects, features, and characteristics of the present disclosure, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of the limits of the invention.

BRIEF DESCRIPTION OF THE DRAWINGS

FIG. 1 is a schematic diagram showing an airway pressure support system according to one particular, non-limiting exemplary embodiment;
FIG. 2 is a flowchart showing a method, not part of the invention, that may be implemented in the pressure support system of FIG. 1 for compensating for the pressure drop across the patient circuit due to the pneumatic resistance thereof according to an exemplary embodiment of the disclosed concept; and
FIG. 3 is a schematic diagram showing an airway pressure support system according to an alternative, non-limiting exemplary embodiment.

DETAILED DESCRIPTION OF EXEMPLARY EMBODIMENTS

As used herein, the singular form of "a", "an", and "the" include plural references unless the context clearly dictates otherwise. As used herein, the statement that two or more parts or components are "coupled" shall mean that the parts are joined or operate together either directly or indirectly, i.e., through one or more intermediate parts or components, so long as a link occurs. As used herein, "directly coupled" means that two elements are directly in contact with each other. As used herein, "fixedly coupled" or "fixed" means that two components are coupled so as to move as one while maintaining a constant orientation relative to each other.

As used herein, the word "unitary" means a component is created as a single piece or unit. That is, a component that includes pieces that are created separately and then coupled together as a unit is not a "unitary" component or body. As employed herein, the statement that two or more parts or components "engage" one another shall mean that the parts exert a force against one another either directly or through one or more intermediate parts or components. As employed herein, the term "number" shall mean one or an integer greater than one (i.e., a plurality).

Directional phrases used herein, such as, for example and without limitation, top, bottom, left, right, upper, lower, front, back, and derivatives thereof, relate to the orientation of the elements shown in the drawings and are not limiting upon the claims unless expressly recited therein.

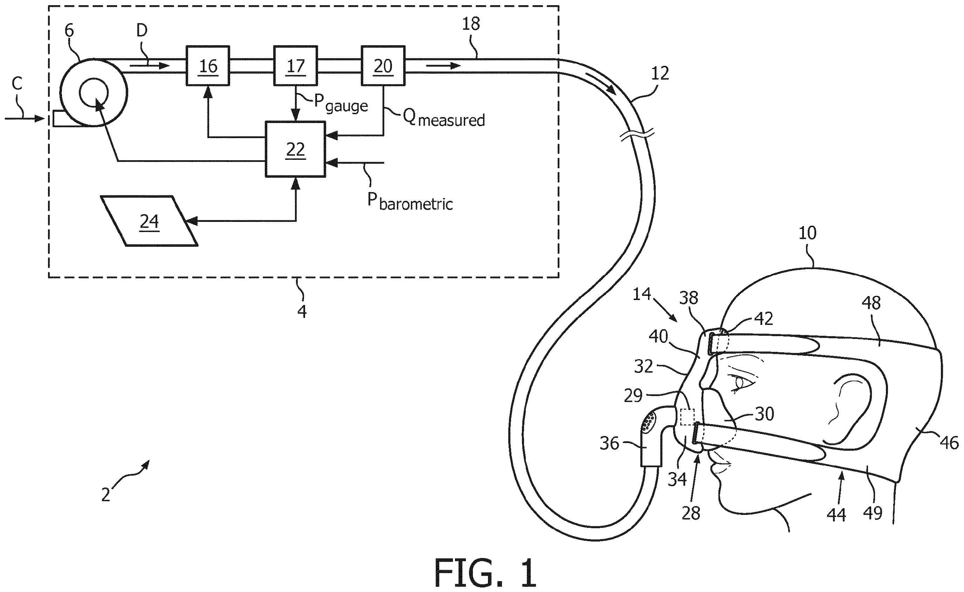

FIG. 1 is a schematic diagram showing an airway pressure support system 2 according to one particular, non-limiting exemplary embodiment. As described in detail herein, pressure support system 2 uses a barometric pressure sensor instead of a gauge pressure sensor to determine the pressure drop over a variable resistance patient circuit, and thus the amount of pressure compensation that is required to overcome the pressure drop due to the variable resistance.

Referring to FIG. 1, airway pressure support system 2 includes a pressure generating device base unit 4 which houses a gas flow generator 6, such as a blower used in a conventional CPAP or bi-level pressure support device. Gas flow generator 6 receives breathing gas, generally indicated by arrow C, from the ambient atmosphere and generates a flow of breathing gas therefrom for delivery to an airway of a patient 10 at relatively higher and lower pressures, i.e., generally equal to or above ambient atmospheric pressure. In the exemplary embodiment, gas flow generator 6 is capable of providing a flow of breathing gas ranging in pressure from 3-30 cmH2O. The pressurized flow of breathing gas from gas flow generator 6, generally indicated by arrow D, is delivered via a delivery conduit 12 to a patient interface device 14 of any known construction, which is typically worn by or otherwise attached to patient 10 to communicate the flow of breathing gas to the airway of patient 10. Delivery conduit 12 and patient interface device 14 are typically collectively referred to as a patient circuit.

Pressure support system 2 shown in FIG. 1 is what is known as a single-limb system, meaning that the patient circuit includes only delivery conduit 12 connecting patient 10 to pressure support system 2. As such, an exhaust vent is provided in patient interface device 14 for venting exhaled gases from the system. It should be noted that the exhaust vent can be provided at other locations in addition to or instead of in patient interface device 14, such as in delivery conduit 12. It should also be understood that the exhaust vent can have a wide variety of configurations depending on the desired manner in which gas is to be vented from pressure support system 2.

The present disclosure also contemplates that pressure support system 2 can be a two-limb system, having a delivery conduit and an exhaust conduit connected to patient 10. In a two-limb system (also referred to as a dual-limb system), the exhaust conduit carries exhaust gas from patient 10 and includes an exhaust valve at the end distal from patient 10. The exhaust valve in such an embodiment is typically actively controlled to maintain a desired level or pressure in the system, which is commonly known as positive end expiratory pressure (PEEP).

In the illustrated embodiment, pressure support system 2 includes a pressure controller in the form of a valve 16 provided in an internal delivery conduit 18 provided in pressure generating device base unit 4 of pressure support system 2. Valve 16 controls the pressure of the flow of breathing gas from gas flow generator 6 that is delivered to patient 10. For present purposes, gas flow generator 6 and valve 16 are collectively referred to as a pressure generating system because they act in concert to control the pressure and/or flow of gas delivered to patient 10. However, it should be apparent that other techniques for controlling the pressure of the gas delivered to patient 10, such as varying the blower speed of gas flow generator 6, either alone or in combination with a pressure control valve, are contemplated by the present disclosure. Thus, valve 16 is optional depending on the technique used to control the pressure of the flow of breathing gas delivered to patient 10. If valve 16 is eliminated, the pressure generating system corresponds to gas flow generator 6 alone, and the pressure of gas in the patient circuit is controlled, for example, by controlling the motor speed of gas flow generator 6.

Pressure support system 2 further includes a gauge pressure sensor 17 that measures the gauge pressure of the flow of breathing gas being output by the pressure generating system, and a flow sensor 20 that measures the flow (i.e., flow rate) of the breathing gas within delivery conduit 18 and delivery conduit 12. In the particular embodiment shown in FIG. 1, pressure sensor 17 and flow sensor 20 are interposed in line with delivery conduit 18, most preferably downstream of valve 16. Gauge pressure sensor 17 generates a pressure signal, $P_{GAUGE}$, that is provided to a controller 22 (described herein). Flow sensor 20 generates a flow signal, $Q_{MEASURED}$, that is provided to controller 22 and is used by controller 22 to determine the flow of gas at patient 10 ($Q_{PATIENT}$). In an alternative implementation, the locations of gauge pressure sensor 17 and flow sensor 20 are reversed from how they are shown in FIG. 1, since flow sensors innately have pressure drop over them, and therefore it may be advantageous to measure pressure at the very outlet of pressure generating device base unit 4.

Techniques for calculating $Q_{PATIENT}$ based on $Q_{MEASURED}$ are well known, and take into consideration the pressure drop of the patient circuit, known leaks from the system, i.e., the intentional exhausting of gas from the circuit as described herein, and unknown (unintentional) leaks from the system, such as leaks at the mask/patient interface. The present disclosure contemplates using any known or hereafter developed technique for calculating total leak flow $Q_{LEAK}$, and using this determination in calculating $Q_{PATIENT}$ based on $Q_{MEASURED}$ (and for other purposes as described elsewhere herein). Examples of such techniques are taught by U.S. Patent Nos. 5,148,802; 5,313,937; 5,433,193; 5,632,269; 5,803,065; 6,029,664; 6,539,940; 6,626,175; and 7,011,091.

Of course, other techniques for measuring and/or estimating the respiratory flow of patient 10 are contemplated by the present disclosure, such as, without limitation, measuring the flow directly at patient 10 or at other locations along delivery conduit 12, measuring patient flow based on the operation of gas flow generator 6, measuring patient flow using a flow sensor upstream of valve 16, and estimating flow based a parameter such as motor current.

Also, pressure at the outlet of pressure generating device base unit 4 can be estimated using a number of methods known to one skilled in the art, including using blower speed, blower speed and barometric pressure, or blower speed, barometric pressure, and outlet flow.

Controller 22 includes a processing portion which may be, for example, a microprocessor, a microcontroller or some other suitable processing device, and a memory portion that may be internal to the processing portion or operatively coupled to the processing portion and that provides a storage medium for data and software executable by the processing portion for controlling the operation of pressure support system 2, including compensating for the pressure drop over the patient circuit based on a barometric pressure measured within the air flow path as described in greater detail herein.

An input/output device 24 is provided for setting various parameters used by airway pressure support system 2, as well as for displaying and outputting information and data to a user, such as a clinician or caregiver.

In the exemplary embodiment, patient interface device 14 includes a patient sealing assembly 28, which in the illustrated embodiment is a nasal mask. However, other types of patient sealing assemblies, such as, without limitation, a nasal/oral mask, a nasal cushion, nasal pillows, a cradle cushion or a full face mask, which facilitate the delivery of the flow of breathing gas to the airway of a patient may be substituted for patient sealing assembly 28 while remaining within the scope of the present disclosure. In the non-limiting exemplary embodiment, patient sealing assembly 28 includes a cushion 30 coupled to a frame member 32. In the illustrated embodiment, cushion 30 is defined from a unitary piece of soft, flexible, cushiony, elastomeric material, such as, without limitation, silicone, an appropriately soft thermoplastic elastomer, a closed cell foam, or any combination of such materials. Also in the illustrated embodiment, frame member 32 is made of a rigid or semi-rigid material, such as, without limitation, an injection molded thermoplastic or silicone, and includes a faceplate portion 34 to which cushion 30 is fluidly attached. A fluid coupling conduit 36 having an exhaust vent is coupled to an opening in faceplate portion 34 to allow the flow of breathing gas from pressure generating device base unit 4 to be communicated to an interior space defined by cushion 30, and then to the airway of a patient. Other options patient sealing assembly 28 are available within the scope of the disclosed concept, such as an implementation wherein the mask does not have a solid frame at all, but instead consists of flexible silicone tubing through which the air is routed from a connection on the crown of the head down two tubes on the side of the face toward the nose.

Frame member 32 also includes a forehead support member 38 that is coupled to the faceplate portion 34 by a connecting member 40. A forehead cushion 42 is coupled to the rear of forehead support member 38. In the exemplary embodiment, forehead cushion 42 is made of a material that is similar to the material of cushion 30.

Patient interface device 14 also includes a headgear component 44 for securing patient interface device 14 to the head of patient 10. Headgear component 44 includes a back member 46, upper strap members 48 and lower strap members 49. In the exemplary embodiment, upper strap members 48 and lower strap members 49 each include a hook and loop fastening system, such as VELCRO®, provided on the end thereof to allow headgear component 44 to be secured in a known manner. It will be understood that the described hook and loop fastening arrangement is meant to be exemplary only, and that other selectively adjustable fastening arrangements are also possible within the scope of the present disclosure.

Pressure support system 2 further includes a barometric pressure sensor 29 that is provided in the airflow path of pressure support system 2 proximate to patient 10. As seen in FIG. 1, in the exemplary embodiment, barometric pressure sensor 29 is shown as being provided inside faceplate portion 34 of patient interface device 14, although it will be understood that this is meant to be exemplary only and that barometric pressure sensor 29 may be positioned in other portions of the airflow path such as within conduit 12 or within other parts of patient interface device 14, such as cushion 30 or fluid coupling conduit 36. Barometric pressure sensor 29 may be any type of known or hereafter developed sensor device suitable for measuring the barometric pressure within the airflow path of pressure support system 2. Barometric pressure sensor 29 is coupled to controller 22 by a suitable wired and/or wireless connection to enable a signal, $P_{BAROMETIC}$, generated by barometric pressure sensor 29 to be communicated to controller 22.

In the illustrated, non-limiting exemplary embodiment of the present disclosure, airway pressure support system 2 essentially functions as a CPAP pressure support system, and, therefore, includes all of the capabilities necessary in such systems in order to provide appropriate CPAP pressure levels to patient 10. This includes receiving the necessary parameters, via input commands, signals, instructions or other information, for providing appropriate CPAP pressure, such as maximum and minimum CPAP pressure settings. It should be understood that this is meant to be exemplary only, and that other pressure support methodologies, including, but not limited to, BiPAP AutoSV, AVAPS, Auto CPAP, and BiPAP Auto, are within the scope of the present disclosure.

As discussed briefly elsewhere herein, the disclosed concept provides a methodology for controlling the pressure generating system (gas flow generator 6 and valve 16 in the illustrated example) of pressure support system 2 in order to compensate for the pneumatic resistance that is present in the patient circuit comprising delivery conduit 12 and patient interface device 14 at any particular time that, rather than being based on a gauge pressure that is measured proximate to patient 10, is based on a barometric pressure that is measured proximate to patient 10. Furthermore, the disclosed concept may be used to estimate either the entire pressure drop of the patient circuit or a portion thereof (i.e., the pressure drop of the patient circuit can contain any of a number of components with known pressure drops and/or known leaks such that a combination of one or more estimated pressure drops (estimated as described herein) and one or more known pressure drops may be employed to determine pressure compensation). For example, in the case of a patient circuit including a flexible headgear portion having a variable resistance tubing positioned after a leak port of the patient circuit, the pressure drop of the variable resistance tubing may be estimated using an estimated/known pressure at the leak port, an estimated patient flow, and the measured barometric pressure.

In particular, according to the disclosed concept, the outlet pressure generated by gas flow generator 6 and output from pressure generating device base unit 4 (Poutlet) is controlled based upon the following equation:

$$\text{Poutlet} = \text{Pptset} + \text{Pdropcalc},$$

wherein Pptset is the desired patient pressure set point (i.e., desired pressure to be delivered to the airway of patient 10 through patient interface device 14) stored by pressure generating device 4, and wherein Pdropcalc is a feedforward term calculated based upon 2 coefficients, A and B (determined as described below) and the total flow, $Q_{MEASURED}$, currently being generated by the pressure generating system. According to an aspect of the disclosed concept, Pdropcalc is determined according to the following equation:

$$\text{Pdropcalc} = A^*Q_{MEASURED}^2 + B^*Q_{MEASURED}.$$

In addition, according to a further aspect of the disclosed concept, the coefficients A and B required for the above calculation may be determined by collecting data and performing modeling of the pressure drop over the patient circuit using certain known equations as described below. In particular, the following two equations are known:

$$\text{Pdiff} = A^*Q_{MEASURED}^2 + B^*Q_{MEASURED} + C,$$

$$\text{Pdrop} = \text{Pdiff} + C,$$

wherein Pdiff is the difference between the gauge pressure of the gas being output by the pressure generating system as measured by gauge pressure sensor 17 and represented by the signal $P_{GAUGE}$ and the barometric pressure in the airflow path at patient interface device 14 as measured by barometric pressure sensor 29 and represented by the signal $P_{BAROMETRIC}$, and wherein Pdrop is the pressure drop over the patient circuit comprising delivery conduit 12 and patient interface device 14. The coefficients A, B and C may derived by collecting and/or determining data comprising the values of $Q_{MEASURED}$, $P_{GAUGE}$, $P_{BAROMETRIC}$, and Pdiff ($P_{GAUGE}$ - $P_{BAROMETRIC}$) over time, generating a Pdiff-$Q_{MEASURED}$ curve based on the data, and deriving the constants A, B and C from the curve fit (e.g., without limitation, least squares estimator (LSE) curve fit) of the Pdiff-$Q_{MEASURED}$ curve.

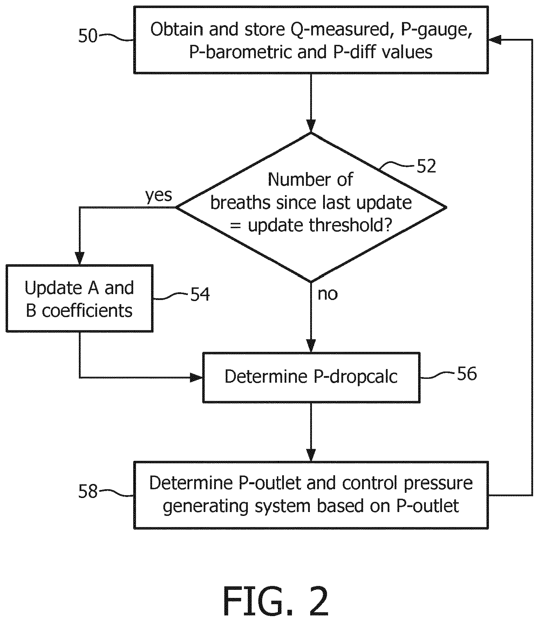

FIG. 2 is a flowchart showing a method that may be implemented in pressure support system 2 for compensating for the pressure drop across the patient circuit due to the pneumatic resistance thereof according to an exemplary embodiment of the disclosed concept. In the exemplary embodiment, the method is implemented in a number of routines stored in the memory of controller 22 and executable by the processor of controller 22. The method of FIG. 2 begins at step 50, wherein current values for $Q_{MEASURED}$, $P_{GAUGE}$, $P_{BAROMETRIC}$, and Pdiff are obtained as described herein and stored. Then, at step 52, a determination is made as to whether the number of breaths since the A and B coefficients has last been updated is equal to a predetermined update threshold. In essence, step 52 ensures that the values of the A and B coefficients get updated periodically, e.g. every breath or every several breaths. If the answer at step 52 is yes, then, at step 54, the A and B coefficients are updated as described elsewhere herein using the collected and stored data. If, however, the answer at step 52 is no, or following the performance of step 54 if appropriate, the method proceeds to step 56. At step 56, the current value for Pdropcalc is determined as described herein (Pdropcalc = $A^*Q_{MEASURED}^2 + BQ_{MEASURED}$) using the updated A and B coefficients and the current $Q_{MEASURED}$ value. Then, at step 58, the value for Poutlet is determined using the stored Pptset and the determined Pdropcalc as described herein (Poutlet = Pptset + Pdropcalc). Also at step 58, once the current value for Poutlet is determined as just described, the pressure generating system of pressure generating device 4 is controlled to output a pressure equal to Poutlet. As will be appreciated, outputting a pressure equal to Poutlet will compensate for the current pneumatic resistance of the patient circuit so as to cause a pressure equal to Pptset to be actually delivered to patient 10.

In alternative embodiments, the outlet pressure of the pressure generating system (Poutlet) may be controlled using the pressure drop equation above using only the square term (i.e., B=0). Furthermore, other governing equations may also be used to determine the pressure drop (e.g., an equation that uses $Q_{MEASURED}$ raised to a different power other than 2).

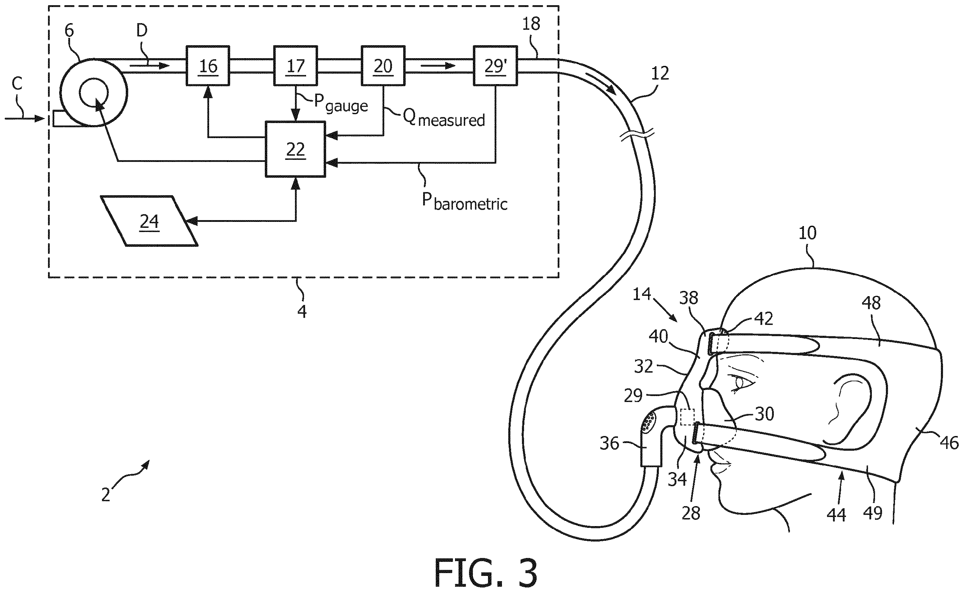

In an alternative embodiment, shown in FIG. 3, the pressure generating device 4 may have a second barometric pressure sensor 29' (in addition to first barometric pressure sensor 29 and gauge pressure sensor 17) for generating a signal $P_{BAROMETRIC}$ indicative of the barometric pressure within the gas flow within pressure generating device base unit 4. In such a case, the gauge pressure being delivered to the patient can be determined by the difference of the two barometric pressure sensor readings, wherein the zero offset between the two barometric pressure sensors can be estimated in the same fashion as described above.

Thus, the disclosed concept provides a system and method wherein the pressure drop in a patient circuit caused by the pneumatic resistance of the patient circuit may be compensated for using a smaller and less expensive barometric pressure sensor as compared to a larger, more expensive gauge pressure sensor proximate the patient.

In the claims, any reference signs placed between parentheses shall not be construed as limiting the claim. The word "comprising" or "including" does not exclude the presence of elements or steps other than those listed in a claim. In a device claim enumerating several means, several of these means may be embodied by one and the same item of hardware. The word "a" or "an" preceding an element does not exclude the presence of a plurality of such elements. In any device claim enumerating several means, several of these means may be embodied by one and the same item of hardware. The mere fact that certain elements are recited in mutually different dependent claims does not indicate that these elements cannot be used in combination.

Although the disclosure has been described in detail for the purpose of illustration based on what is currently considered to be the most practical and preferred embodiments, it is to be understood that such detail is solely for that purpose and that the disclosure is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

**Claims**

1. A pressure support system (2) for delivering a flow of breathing gas to an airway of a patient (10), comprising:

   a pressure generating system (6, 16) structured to generate the flow of breathing gas;
   a patient circuit including a patient interface device (14), the patient circuit being coupled to the pressure generating system and structured to carry the flow of breathing gas;
   a barometric pressure sensor (29) coupled to the patient circuit within a gas flow path of the patient circuit, wherein the barometric pressure sensor is structured to generate a barometric pressure signal indicative of a barometric pressure within the gas flow path proximate the patient; and
   a control system (22) structured to control an outlet pressure generated by the pressure generating system based on at least the barometric pressure signal in a manner that compensates for a pressure drop across the patient circuit due to a pneumatic resistance of the patient circuit, **characterized in that** the barometric pressure sensor is directly connected to the patient interface device.

2. The pressure support system according to claim 1, wherein the pressure generating system is provided within a pressure generating base unit (4), wherein the pressure generating base unit includes a flow sensor (20) structured to generate a flow signal ($Q_{MEASURED}$) indicative of a total flow rate being generated by the pressure generating system and a base unit pressure sensor (17) structured to generate a base unit pressure signal, and wherein the control system is structured to control the outlet pressure based on the barometric pressure signal, the flow signal and the base unit pressure signal in a manner that compensates for the pressure drop across the patient circuit due to the pneumatic resistance of the patient circuit.

3. The pressure support system according to claim 2, wherein the base unit pressure sensor is a gauge pressure sensor and wherein the base unit pressure signal is a gauge pressure signal indicative of a gauge pressure being generated by the pressure generating system.

4. The pressure support system according to claim 3, wherein the flow signal is $Q_{MEASURED}$, the barometric pressure signal is $P_{BAROMETRIC}$, and the gauge pressure signal is $P_{GAUGE}$, wherein the control system is structured to determine the outlet pressure (Poutlet) as a function of $Q_{MEASURED}$, $P_{BAROMETRIC}$, and $P_{GAUGE}$.

5. The pressure support system according to claim 4, wherein the control system is structured to determine the outlet pressure (Poutlet) according to the following equation: Poutlet = Pptset + Pdropcalc, wherein Pptset is a desired patient pressure set point, wherein Pdropcalc = $A^*Q_{MEASURED}^2 + B^*Q_{MEASURED}$, and wherein A and B are coefficients derived using a plurality of collected and stored values of $Q_{MEASURED}$, $P_{BAROMETRIC}$, and $P_{GAUGE}$.

6. The pressure support system according to claim 5, wherein Pdiff is a difference between $P_{GAUGE}$ and $P_{BAROMETRIC}$, wherein A and B are derived by generating a Pdff-$Q_{MEASURED}$ curve using the collected and stored values of $Q_{MEASURED}$, $P_{BAROMETRIC}$, and $P_{GAUGE}$ and deriving A and B from a curve fit of the Pdff-$Q_{MEASURED}$ curve.

7. The pressure support system according to claim 6, wherein the curve fit is a least squares estimator curve fit.

8. The pressure support system according to claim 6, wherein the controller is structured to update A and B periodically.

9. The pressure support system according to claim 1, wherein the pressure generating system is provided within a pressure generating base unit (4), the pressure support system further comprising a second barometric pressure sensor (29') provided within the pressure generating base unit and within a gas flow path of the pressure generating base unit, wherein the second barometric pressure sensor is structured to generate a second barometric pressure signal indicative of a barometric pressure within the pressure generating base unit, and wherein the control system is structured to control the outlet pressure generated by the pressure generating system based on at least the barometric pressure signal and the second barometric pressure signal in a manner that compensates for a pressure drop across the patient circuit due to a pneumatic resistance of the patient circuit

**Patentansprüche**

1. Druckunterstützungssystem (2) zum Zuführen eines Atemgasstroms zu einem Atemweg eines Patienten (10), umfassend:

ein Druckerzeugungssystem (6, 16), das strukturiert ist, um den Atemgasstrom zu erzeugen; einen Patientenkreislauf mit einer Patientenschnittstellenvorrichtung (14), wobei der Patientenkreislauf mit dem Druckerzeugungssystem gekoppelt und strukturiert ist, um den Atemgasstrom zu führen; einen Luftdrucksensor (29), der mit dem Patientenkreislauf innerhalb eines Gasströmungswegs des Patientenkreislaufes gekoppelt ist, wobei der Luftdrucksensor strukturiert ist, um ein Luftdrucksignal zu erzeugen, das einen Luftdruck innerhalb des Gasströmungswegs in der Nähe des Patienten anzeigt; und

ein Steuersystem (22), das strukturiert ist, um einen von dem Druckerzeugungssystem erzeugten Auslassdruck basierend auf mindestens dem Luftdrucksignal auf eine Weise zu steuern, die einen Druckabfall über den Patientenkreislauf aufgrund eines pneumatischen Widerstands des Patientenkreislaufs kompensiert, **dadurch gekennzeichnet, dass** der Luftdrucksensor direkt mit der Patientenschnittstelle verbunden ist.

2. Druckunterstützungssystem nach Anspruch 1, wobei das Druckerzeugungssystem innerhalb einer Druckerzeugungsbasiseinheit (4) vorgesehen ist, wobei die Druckerzeugungsbasiseinheit einen Durchflusssensor (20) umfasst, der strukturiert ist, um ein Durchflusssignal ($Q_{MEASURED}$) zu erzeugen, der eine Gesamtströmungsrate anzeigt, die durch das Druckerzeugungssystem erzeugt wird, und einen Basiseinheits-Drucksensor (17) umfasst, der strukturiert ist, um ein Basiseinheit-Drucksignal zu erzeugen, und wobei das Steuersystem strukturiert ist, um den Auslassdruck basierend auf dem Luftdrucksignal, dem Durchflusssignal und dem Drucksignal der Basiseinheit auf eine Weise zu steuern, die den Druckabfall im Patientenkreislauf aufgrund des pneumatischen Widerstands des Patientenkreislaufs kompensiert.

3. Druckunterstützungssystem nach Anspruch 2, wobei der Basiseinheits-Drucksensor ein Überdrucksensor ist und wobei das Basiseinheits-Drucksignal ein Überdrucksignal ist, das einen Überdruck anzeigt, der durch das Druckerzeugungssystem erzeugt wird.

4. Druckunterstützungssystem nach Anspruch 3, wobei das Durchflusssignal $Q_{MEASURED}$ ist, das Luftdrucksignal $P_{BAROMETRIC}$ ist und das Überdrucksignal $P_{GAUGE}$ ist, wobei das Steuersystem strukturiert ist, um den Auslassdruck (Poutlet) als Funktion von $Q_{MEASURED}$, $P_{BAROMETRIC}$ und $P_{GAUGE}$ zu bestimmen.

5. Druckunterstützungssystem nach Anspruch 4, wobei das Steuersystem strukturiert ist, um den Auslassdruck (Poutlet) gemäß der folgenden Gleichung zu bestimmen: Poutlet = Pptset + Pdropcalc, wobei Pptset ein gewünschter Patientendrucksollwert ist, wobei Pdropcalc = $A*Q_{MEASURED}^2 + B*Q_{MEASURED}$, und wobei A und B Koeffizienten sind, die unter Verwendung einer Vielzahl von gesammelten und gespeicherten Werten von $Q_{MEASURED}$, $P_{BAROMETRIC}$ und $P_{GAUGE}$ abgeleitet werden.

6. Druckunterstützungssystem nach Anspruch 5, wobei Pdiff eine Differenz zwischen $P_{GAUGE}$ und $P_{BAROMETRIC}$ ist, wobei A und B abgeleitet werden durch Erzeugen einer Pdff-$Q_{MEASURED}$-Kurve unter Verwendung der gesammelten und gespeicherten Werte von $Q_{MEASURED}$, $P_{BAROMETRIC}$ und $P_{GAUGE}$ und Ableiten von A und B aus einer Kurvenanpassung der Pdff-$Q_{MEASURED}$-Kurve.

7. Druckunterstützungssystem nach Anspruch 6, wobei die Kurvenanpassung eine Kurvenanpassung nach der Methode der kleinsten Quadrate ist.

8. Druckunterstützungssystem nach Anspruch 6, wobei die Steuerung strukturiert ist, um A und B periodisch zu aktualisieren.

9. Druckunterstützungssystem nach Anspruch 1, wobei das Druckerzeugungssystem innerhalb einer druckerzeugenden Basiseinheit (4) vorgesehen ist, wobei das Druckunterstützungssystem ferner einen zweiten Luftdrucksensor (29') umfasst, der innerhalb der druckerzeugenden Basiseinheit und innerhalb eines Gasströmungswegs der druckerzeugenden Basiseinheit vorgesehen ist, wobei der zweite Luftdrucksensor strukturiert ist, um ein zweites Luftruck signal zu erzeugen, das einen Luftdruck innerhalb der druckerzeugenden Basiseinheit anzeigt, und wobei das Steuersystem strukturiert ist, um den Aus lassdruck, der von dem Druckerzeugungssystem basierend auf mindestens dem Luftdrucksignal und dem zweiten Luftdrucksignal in einer Weise erzeugt wird, die einen Druckabfall über den Patientenkreis lauf aufgrund eines pneumatischen Widerstands des Patientenkreislaufs kompensiert

**Revendications**

1. Système de support de pression (2) pour délivrer un flux de gaz respiratoire à une voie aérienne d'un patient (10), comprenant:

un système de génération de pression (6, 16) structuré pour générer le flux de gaz respiratoire; un circuit patient comprenant un dispositif d'interface patient (14), le circuit patient étant couplé au système de génération de pression et structuré pour transporter le flux de gaz respiratoire; un capteur de pression barométrique (29) couplé au circuit patient dans un trajet d'écoulement de gaz du circuit patient, où le capteur de pression barométrique est structuré pour générer un signal de pression barométrique indicatif d'une pression barométrique dans le trajet d'écoulement du gaz à proximité du patient; et un système de commande (22) structuré pour commander une pression de sortie générée par le système de génération de pression sur la base d'au moins le signal de pression barométrique d'une manière qui compense une chute de pression dans le circuit patient due à une résistance pneumatique du circuit patient, **caractérisé en ce que** le capteur de pression barométrique est directement connecté au dispositif d'interface patient.

2. Système de support de pression selon la revendication 1, dans lequel le système de génération de la pression est fourni dans une unité de base de génération de pression (4), où l'unité de base de génération de pression comprend un capteur de flux (20) structuré pour générer un signal de flux ($Q_{MEASURED}$) indicatif d'un débit total qui est généré par le système de génération de pression et un capteur de pression de l'unité de base (17) structuré pour générer un signal de pression de l'unité de base, et dans lequel le système de commande est structuré pour commander la pression de sortie sur la base du signal de pression barométrique, du signal de flux et du signal de pression de l'unité de base d'une manière qui compense la chute de pression dans le circuit patient due à la résistance pneumatique du circuit patient.

3. Système de support de pression selon la revendication 2, dans lequel le capteur de pression de l'unité de base est un capteur de pression manométrique et dans lequel le signal de pression de l'unité de base est un signal de pression manométrique indicatif d'une pression manométrique qui est générée par le système de génération de pression.

4. Système de support de pression selon la revendication 3, dans lequel le signal de flux est $Q_{MEASURED}$, le signal de pression barométrique est $P_{BAROMETRIC}$, et le signal de pression manométrique est $P_{GAUGE}$, où le système de commande est structuré pour déterminer la pression de sortie (Poutlet) en fonction de $Q_{MEASURED}$, $P_{BAROMETRIC}$, et $P_{GAUGE}$.

5. Système de support de pression selon la revendication 4, dans lequel le système de commande est structuré pour déterminer la pression de sortie (Poutlet) selon l'équation suivante: Poutlet = Pptset + Pdropcalc, où Pptset est un point de consigne de pression du patient souhaité, où Pdropcalc = $A*Q_{MEASURED}^2 + B*Q_{MEASURED}$, et où A et B sont des coefficients dérivés en utilisant une pluralité de valeurs collectées et stockées de $Q_{MEASURED}$, $P_{BAROMETRIC}$ et $P_{GAUGE}$.

6. Système de support de pression selon la revendication 5, dans lequel Pdiff est une différence entre $P_{GAUGE}$ et $P_{BAROMETRIC}$, où A et B sont dérivés en générant une courbe Pdff-$Q_{MEASURED}$ en utilisant les valeurs collectées et stockées de $Q_{MEASURED}$, $P_{BAROMETRIC}$, et $P_{GAUGE}$ et en dérivant A et B à partir d'un ajustement de courbe de la courbe Pdff-

Q<sub>MEASURED</sub>.

**7.** Système de support de pression selon la revendication 6, dans lequel l'ajustement de courbe est un ajustement de courbe d'estimateur des moindres carrés.

**8.** Système de support de pression selon la revendication 6, dans lequel l'unité de commande est structurée pour mettre à jour A et B périodiquement.

**9.** Système de support de pression selon la revendication 1, dans lequel le système de génération de pression est fourni dans une unité de base de génération de pression (4), le système de support de pression comprenant en outre un second capteur de pression barométrique (29') fourni dans l'unité de base de génération de pression et dans un trajet d'écoulement de gaz de l'unité de base de génération de pression, où le second capteur de pression barométrique est structuré pour générer un second signal de pression barométrique indicatif d'une pression barométrique dans l'unité de base de génération de pression, et où le système de commande est structuré pour commander la pression de sortie générée par le système de génération de pression sur la base d'au moins le signal de pression barométrique et le second signal de pression barométrique d'une manière qui compense une chute de pression dans le circuit patient due à une résistance pneumatique du circuit patient.

FIG. 1

50 — Obtain and store Q-measured, P-gauge,
P-barometric and P-diff values

52
Number of
breaths since last update
= update threshold?

yes

no

Update A and
B coefficients — 54

Determine P-dropcalc — 56

58 — Determine P-outlet and control pressure
generating system based on P-outlet

# FIG. 2

FIG. 3

EP 3 316 949 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2013056006 A
- WO 2014057457 A
- US 2009241952 A
- US 2012227738 A
- US 2010078023 A
- US 5148802 A
- US 5313937 A
- US 5433193 A
- US 5632269 A
- US 5803065 A
- US 6029664 A
- US 6539940 B
- US 6626175 B
- US 7011091 B